# DEMANDE DE BREVET EUROPEEN

(11) **EP 4 094 677 A1**
(43) Date de publication de la demande: **30.11.2022**
(21) Numéro de dépôt: 21305703.7
(22) Date de dépôt: 27.05.2021
(51) Int. Cl.: A61B 5/00, A61B 8/00, H04N 7/08, G16H 10/00, G16H 80/00

(54) **STATION PATIENT POUR LA TELEMEDECINE**

(71) Demandeur: Hopi Medical, 67560 Rosheim (FR)
(72) Inventeur: UHLRICH, Damien, 54500 Vandoeuvre-les-Nancy (FR)
(74) Mandataire: Osha Liang

(57) **Abrégé**

Station patient (100) pour la télémédecine permettant d'acquérir des données relatives à un patient et de transmettre ces données vers une station médecin (30) distante, via un réseau de télécommunication (20), la station patient (100) comprenant : une unité de traitement informatique (110), un dispositif d'affichage principal (120), et au moins un premier capteur (150) générant des premières données patient (151). L'unité de traitement (110) est configurée pour : générer un premier signal vidéo (153) ou une première image à partir des premières données patient (151) et afficher le premier signal vidéo (153) ou la première image sur le dispositif d'affichage principal dans une première fenêtre d'affichage (140) ; capturer au moins une zone d'affichage (145) de la première fenêtre d'affichage (140) pour générer un signal vidéo capturé (155) ; et transmettre le signal vidéo de sortie (157) comprenant le signal vidéo capturé (155) vers la station médecin (10) via le réseau de télécommunication (20).

## Description

### DOMAINE TECHNIQUE

La présente invention se rapporte au domaine technique général de l'électronique et de l'informatique appliquée à la médecine et plus particulièrement à la télémédecine.

### ARRIERE PLAN

La consultation est le fondement de la pratique médicale. Elle suppose la présence simultanée, sur un même site, des deux protagonistes, le patient demandeur de soins et le praticien prodiguant des conseils, des prescriptions ou des soins. Dans la présente demande, ce praticien est appelé "médecin", sans que cela n'implique une quelconque limitation quant à sa formation, ses diplômes ou la nature des soins prodigués. Typiquement, le médecin réalise une prestation résultant idéalement en un diagnostic, un pronostic et une proposition thérapeutique. Cette consultation peut se compléter, dans la même séance ou de façon différée, d'examens de biologie, d'imagerie ou autres apportant des informations complémentaires. Le médecin pourra aussi demander l'avis éclairé de collègues experts, le tout visant à étayer et à consolider son diagnostic.

La téléconsultation est la réalisation de cette prestation à distance lorsque le patient et le médecin ne sont pas physiquement en présence dans la même pièce, mais sont séparés. Par exemple, les deux acteurs peuvent être séparés d'une distance de quelques mètres lorsqu'ils se trouvent dans deux pièces adjacentes ou proches non communicantes, jusqu'à des distances de plusieurs milliers de kilomètres.

Toute consultation médicale a pour objectif principal de parvenir à un diagnostic. Dans sa vie professionnelle, un médecin pratique environ 200 000 consultations. Près de 7 fois sur 10, il parvient à cerner le diagnostic dès la première rencontre qui sera confirmé par d'autres investigations éventuelles et par l'évolution sous traitement. Dans les 30% restant, il fera appel à des compétences extérieures sous forme d'examens complémentaires, d'avis de collègues spécialistes sous la forme d'échanges bilatéraux ou de réunions élargies de concertations pluridisciplinaires. Un ou plusieurs séjours hospitaliers peuvent s'avérer nécessaires dans les cas les plus complexes.

Toute consultation médicale comporte généralement les mêmes étapes qui se décomposent ainsi : le médecin accueille le patient, il l'écoute, lui pose des questions ciblées qui structurent la démarche diagnostique, il enregistre ses réponses et observe son comportement et d'éventuels faits saillants.

Après cette étape initiale, purement conversationnelle, près d'une fois sur deux il tient le diagnostic qu'il s'attachera à partir de là, à confirmer. Il appréciera le degré de retentissement des troubles sur l'individu ce qui est lié à la gravité de l'affection mais aussi à la personnalité du patient et à son ressenti subjectif.

Il recherche alors des éléments objectifs accessibles à l'examen clinique dans le cabinet médical, par exemple par :
- l'inspection précise d'éléments ou de zones indiquées par le patient ou découvertes par le médecin,
- la palpation de confirmation, réveillant une douleur ou induisant une réaction musculaire ou d'exploration à la recherche de symptômes non spontanément perçus par le patient,
- la percussion qui a pour objet de différencier les zones solides, les liquides et les épanchements gazeux,
- l'auscultation enfin, cardio-pulmonaire principalement, mais aussi sur le trajet des vaisseaux, sur l'abdomen à la recherche d'un souffle, de bruits divers ou, à l'inverse, de silences.

A distance, la consultation qui devient téléconsultation, nécessite la mise en œuvre de moyens techniques pour que le médecin puisse la réaliser complètement. En général la téléconsultation consiste alors à obtenir des données relatives au patient, appelées "données patient", et à transmettre ces données au médecin. Les données patient sont recueillies sur le site où se trouve le patient, appelé "site patient", par des instruments d'observation et/ou de mesure et des moyens informatiques qui forment un ensemble communément appelé "station patient". Ces données sont transmises par la station patient vers le poste de travail du médecin situé sur un "site médecin" distant, via un réseau de télécommunications. Le poste de travail du médecin est équipé de moyens informatiques et est communément appelé "station médecin".

Pour la phase conversationnelle, un dispositif comprenant un logiciel de vidéoconférence permet d'arriver au but.

Pour l'examen clinique, il s'agit d'utiliser des capteurs capables de transmettre à distance le/les signaux qu'ils génèrent pour que le médecin puisse les recevoir et les interpréter en direct. Des capteurs connectables à une unité de traitement proche du patient qui assure une transmission des signaux est également une approche technique qui fonctionne.

A titre d'exemple, toujours pour l'examen clinique, on peut citer pour chacune des quatre étapes les capteurs connectables suivants :
- le dermatoscope, l'otoscope, le laryngoscope, l'endoscope, la caméra mobile autonome, le rétinographe, la lampe à fente, etc. pour l'étape de l'inspection.
- l'échographe pour l'étape de la palpation est possible en demandant à l'assistant de déplacer la sonde sur la zone à examiner. Outre les images produites, le médecin peut notamment regarder au niveau du visage du patient, les réactions produites au cours de l'examen pour relever une grimace de douleur, un regard, un gémissement, etc... On notera à ce stade qu'un double signal est nécessaire pour le médecin : celui de la sonde échographique et celui de la vidéo de la scène générale afin que ce dernier puisse observer les réactions du patient.
- l'échographe également pour l'étape de la percussion trouve une réponse immédiate puisque le médecin pourra différencier l'image noire et homogène du liquide, l'aspect hétérogène des solides et l'arrêt immédiat de la transmission des ultrasons par les gaz.
- Le stéthoscope pour l'étape de l'auscultation avec un traitement possible du signal sonore pour amplifier ou atténuer certaine fréquence.

Pour des examens plus spécialisés, on peut également citer, par exemple, l'électrocardiogramme, l'électroencéphalogramme, l'échographe doppler, l'échographe doppler cardiaque.

En 2021, en France, il existe plus d'une centaine de solutions logicielles de téléconsultation, ou plateformes, éditées, développées et/ou gérées par des entités privées, publiques ou parapubliques.

Cependant, ces plateformes n'ont pas toutes la même richesse fonctionnelle concernant notamment la complexité de l'examen clinique à distance qu'il est possible de réaliser. La richesse fonctionnelle est principalement liée à la variété de capteurs différents pouvant être utilisés avec la plateforme. Pour certaines plateformes comprenant peu de fonctionnalités et donc compatible avec peu de capteurs, il s'agit souvent d'un choix industriel décidé par l'éditeur qui consiste à ne pas développer ou utiliser de dispositifs électroniques et de capteurs côté patient. Ainsi un examen requérant un certain type de capteur peut imposer le choix d'une plateforme particulière au médecin. De plus, aucune de ces plateformes n'est interopérable, ce qui veut dire qu'un médecin inscrit chez un éditeur ne peut pas réaliser de téléconsultation pour un patient inscrit chez un éditeur différent.

Dans la plupart des cas, les plateformes proposent au moins une fonction de vidéoconférence permettant la réalisation de la phase conversationnelle de la téléconsultation. Cette fonction de vidéoconférence (assurée par un logiciel indépendant et autonome ou intégrée à un navigateur internet) permet au plus la transmission simultanée d'un signal vidéo et d'un signal audio.

Partant de ce constat, les inventeurs ont eu l'idée d'utiliser uniquement la fonction de vidéoconférence d'une plateforme de téléconsultation (ou d'une plateforme de vidéoconférence classique dont tout le monde peut disposer aisément de nos jours) afin de réaliser la téléconsultation et transmettre les données patient vers la station médecin. Ils ont toutefois rapidement été confrontés aux problèmes suivants.

Lors d'une téléconsultation, il peut être nécessaire d'utiliser différents capteurs produisant des données de différents types qui ne sont pas toutes transmissibles par vidéoconférence. En outre, il peut être nécessaire de transmettre plusieurs signaux simultanément, par exemple un signal correspondant à un électrocardiogramme, et un autre correspondant à une image du patient captée par une webcam, ce que ne permet pas une plateforme de vidéoconférence classique.

De plus, certains capteurs sont fournis par un fabricant avec un logiciel imposé qui permet d'afficher les données issues du capteur dans une interface graphique dédiée, mais ne permet pas d'accéder aux données pour les transmettre via une plateforme de type vidéoconférence. Ainsi, dans certains cas, les données issues du capteur sont affichées sur la station patient mais ne peuvent pas être transférées vers la station médecin. Dans d'autres cas, le transfert est possible mais requiert l'utilisation d'une plateforme particulière non-interopérable avec d'autres plateformes.

Plus généralement, la "non-interopérabilité" des plateformes provoque, entre autres, les problèmes suivants :
- Une segmentation de l'offre médicale pour le patient. Selon la plateforme sur laquelle il est inscrit ou à laquelle il a accès, il ne pourra pas accéder au médecin de son choix. Ceci est d'autant plus vrai si ce dernier réside en établissement d'hébergement spécialisé et/ou que son établissement à fait le choix d'une solution de téléconsultation unique.
- Une segmentation de la patientèle pour le médecin. Il ne pourra pas répondre à tous ses patients. S'il s'inscrit sur plusieurs plateformes, il ne peut pas réaliser de prestations médicales à qualité équivalente car ces dernières dépendent de la richesse fonctionnelle de chacune d'entre elles.

L'objet de la présente invention est donc de pallier, au moins en partie, certains des problèmes précités. En particulier, la présente invention vise à proposer une station patient compatible avec la plupart des plateformes de téléconsultation pouvant être utilisées dans une station médecin.

### PRESENTATION GENERALE

Selon un premier aspect, la présente invention concerne une station patient pour la télémédecine permettant d'acquérir des données relatives à un patient et de transmettre ces données vers une station médecin distante, via un réseau de télécommunication, la station patient comprenant :
- une unité de traitement informatique ;
- un dispositif d'affichage principal ; et
- au moins un premier capteur d'un premier type générant des premières données patient.
L'unité de traitement est configurée pour :
- générer un premier signal vidéo ou une première image à partir des premières données patient et afficher le premier signal vidéo ou la première image sur le dispositif d'affichage principal dans une première fenêtre d'affichage ;
- capturer au moins une zone d'affichage de la première fenêtre d'affichage pour générer un signal vidéo capturé ; et
- transmettre un signal vidéo de sortie comprenant le signal vidéo capturé vers la station médecin via le réseau de télécommunication.

Une telle configuration permet d'envoyer vers la station médecin un signal vidéo de sortie représentatif de données patient issues de tous types de capteurs, y compris d'un capteur dont les données sont affichables, sous forme d'image ou de vidéo, uniquement dans une interface graphique dédiée au capteur. En particulier, il est possible de sélectionner, au moyen de la fonction de capture, également appelée capture d'écran (en anglais "screen capture"), une zone d'affichage dans la première fenêtre d'affichage pour extraire et transmettre tout ou partie du premier signal vidéo ou de la première image vers la station médecin distante, et ce quel que soit le logiciel et l'interface imposés par le constructeur du capteur pour afficher le premier signal vidéo ou la première image. Une telle configuration permet donc d'utiliser une grande variété de capteurs lors d'une téléconsultation.

En outre, une telle station patient permet de transmettre des données patient vers n'importe quelle station médecin distante du moment que cette station médecin soit configurée pour recevoir un signal vidéo. En particulier, cette station patient est compatible avec n'importe quelle station médecin équipée d'un logiciel de téléconsultation doté d'une fonction de vidéoconférence ou d'un "simple" logiciel de vidéoconférence. Cette station patient est donc compatible avec la quasi-totalité, si ce n'est la totalité, des stations médecins existantes.

La station patient de l'invention permet donc de transmettre à la quasi totalité des stations médecins une grande variété de données patient issues de différents types de capteurs. En d'autres termes, grâce à cette station patient, un médecin a la possibilité de réaliser un examen clinique complexe basé sur différentes données patient, quel que soit le logiciel de téléconsultation qu'il utilise, voire en utilisant un "simple" logiciel de vidéoconférence dont la plupart des patients et médecins peuvent disposer.

On notera que le signal vidéo de sortie peut comprendre uniquement le signal vidéo capturé, auquel cas ces deux signaux sont identiques, ou peut comprendre un ou plusieurs autres signaux en plus du signal vidéo capturé.

Dans certains modes de réalisation, la station patient comprend, en outre, au moins un deuxième capteur d'un deuxième type, différent du premier type, générant des deuxièmes données patient, et l'unité de traitement est configurée pour :
- générer un deuxième signal vidéo à partir des deuxièmes données patient ; et
- générer le signal vidéo de sortie par combinaison du deuxième signal vidéo et du signal vidéo capturé.

Une telle configuration permet l'utilisation simultanée de plusieurs capteurs pendant la téléconsultation et une transmission simultanée des signaux vidéos qui en sont issus vers la station médecin.

En particulier, la combinaison du deuxième signal vidéo et du signal vidéo capturé en un unique signal vidéo combiné permet d'utiliser un logiciel de vidéoconférence classique qui ne permet la transmission que d'un seul signal vidéo (et d'un seul signal audio).

Dans certains modes de réalisation, l'unité de traitement est associée à un système d'exploitation et est configurée pour que le signal vidéo de sortie soit identifié par le système d'exploitation comme un signal vidéo émanant d'une caméra numérique connectée à l'unité de traitement.

Une telle configuration permet de faire reconnaître en tant que signal vidéo "normal" un signal qui provient d'une capture d'une zone d'affichage. Cette fonctionnalité peut être réalisée au moyen d'un logiciel de type pilote de périphérique (en anglais "driver") qui indique au système d'exploitation que le signal vidéo de sortie provient d'un périphérique de type caméra numérique, comme une webcam, alors que ce périphérique n'existe pas en réalité (i.e. ce périphérique est "virtuel"). Le signal vidéo de sortie va ainsi se retrouver référencé par le système d'exploitation et, par là même, par les logiciels installés sur l'unité de traitement, parmi les signaux vidéo émanant des caméras numériques connectées à l'unité de traitement.

En particulier, dans certains modes de réalisation, la transmission du signal vidéo de sortie vers la station médecin est réalisée par l'intermédiaire d'un logiciel de vidéoconférence. L'unité de traitement est alors configurée pour que le signal vidéo de sortie soit identifié par le logiciel de vidéoconférence comme un signal vidéo émanant d'une caméra numérique connectée à l'unité de traitement.

Une telle configuration permet de choisir le signal vidéo de sortie (qui provient d'une capture d'une zone d'affichage) comme signal utilisé par le logiciel de vidéoconférence et transmis vers le réseau en executant ce logiciel. En pratique, le signal vidéo de sortie peut être sélectionné par l'utilisateur de la station patient dans le menu déroulant de l'interface du logiciel de vidéoconférence qui liste les signaux vidéo disponibles.

Dans certains modes de réalisation, la station patient comprend un capteur audio relié à l'unité de traitement et générant un premier signal audio, et l'unité de traitement est configurée pour que le premier signal audio soit identifié par le système d'exploitation comme un signal audio émanant d'un microphone connecté à l'unité de traitement.

Il est ainsi possible de faire reconnaître en tant que signal audio "normal" un premier signal audio provenant d'un capteur audio particulier comme, par exemple, un stéthoscope. Le premier signal audio se retrouve ainsi référencé par le système d'exploitation et, par là même, par les logiciels installés sur l'unité de traitement, parmi les signaux audio émanant des microphones connectés à l'unité de traitement.

En particulier, dans certains modes de réalisation, le premier signal audio est transmis vers la station médecin par l'intermédiaire d'un logiciel de vidéoconférence. L'unité de traitement est alors configurée pour que le premier signal audio soit identifié par le logiciel de vidéoconférence comme un signal audio émanant d'un microphone connecté à l'unité de traitement.

Une telle configuration permet de choisir le premier signal audio comme signal utilisé par le logiciel de vidéoconférence et transmis vers le réseau en executant ce logiciel. En pratique, le premier signal audio peut être sélectionné par l'utilisateur de la station patient dans le menu déroulant de l'interface du logiciel de vidéoconférence qui liste les signaux audio disponibles.

Dans certains modes de réalisation, l'unité de traitement est configurée pour afficher la première fenêtre d'affichage à l'intérieur d'une deuxième fenêtre d'affichage d'une interface graphique de contrôle.

Une telle configuration permet, en particulier, de contrôler la position et/ou la taille de la première fenêtre d'affichage comprenant le premier signal vidéo issu du premier capteur en contrôlant la position et/ou la taille de la deuxième fenêtre d'affichage.

Dans certains modes de réalisation, l'unité de traitement est configurée pour redimensionner automatiquement la première fenêtre d'affichage affichée dans l'interface graphique de contrôle.

Une telle configuration permet, lorsqu'une troisième fenêtre d'affichage comprenant un signal vidéo ou une image issu d'un autre capteur est affichée dans la deuxième fenêtre d'affichage, de redimensionner la première fenêtre d'affichage afin de rendre visible simultanément l'intégralité de la première fenêtre d'affichage et de la troisième fenêtre d'affichage au sein de la deuxième fenêtre d'affichage.

En particulier, cela permet, lors de de l'affichage (sur le dispositif d'affichage de la station patient) d'un signal vidéo provenant d'une webcam de la station medecin et permettant au patient de voir le médecin, et de l'affichage d'un signal vidéo (ou d'une image) issu d'un capteur mesurant et générant des données patient, de redimensionner la première fenêtre affichant le signal vidéo du capteur par rapport à la fenêtre affichant le signal vidéo du médecin. Il est ainsi possible de voir le médecin tout en effectuant une capture d'une zone d'affichage de la première fenêtre.

Dans certains modes de réalisation, la première fenêtre d'affichage fait partie d'une interface graphique dédiée au premier capteur. C'est notamment le cas lorsque le premier capteur émane d'un fabricant imposant un logiciel pour afficher dans une interface graphique dédiée les données issues du capteur. Typiquement, outre le première fenêtre d'affichage, l'interface graphique comprend des menus, pictogrammes, etc. dédiés au contrôle du premier capteur.

Dans certains modes de réalisation, l'unité de traitement comprend une unité principale et une unité auxiliaire, l'unité auxiliaire étant configurée pour générer le premier signal vidéo ou la première image à partir des premières données patient et afficher le premier signal vidéo ou la première image sur un dispositif d'affichage auxiliaire ; et l'unité principale étant configurée pour dupliquer l'affichage du premier signal vidéo ou de la première image sur le dispositif d'affichage principal dans la première fenêtre d'affichage.

Une telle configuration permet l'utilisation d'une unité auxiliaire pour la génération du premier signal vidéo (ou de la première image) qui est distincte de l'unité principale configurée et utilisée pour la transmission du signal vidéo de sortie vers la station médecin.

En particulier, il est possible d'effectuer une téléconsultation comprenant l'utilisation d'un capteur nécessitant une unité auxiliaire particulière qui n'est pas l'unité principale. Ceci est particulièrement avantageux lorsque un capteur nécessite l'utilisation d'une tablette électronique ou d'un téléphone multifonction (ou smartphone), constituant l'unité auxiliaire, alors que l'appplication de vidéoconférence utilisée pour la transmission des signaux vidéo est exécutée sur un ordinateur personnel (ou PC) constituant l'unité principale.

Dans certains modes de réalisation, la génération du signal vidéo de sortie comprend une étape de filtrage et/ou de redimensionnement du signal vidéo capturé et/ou du deuxième signal vidéo.

Une telle configuration permet de modifier des paramètres du signal vidéo capturé et/ou du deuxième signal vidéo avant de les combiner pour générer le signal vidéo de sortie. En particulier, cela permet de modifier la résolution et/ou les dimensions du signal vidéo capturé et du deuxième signal vidéo en une résolution commune (et/ou des dimensions communes) afin de générer un signal vidéo de sortie ayant des paramètres homogènes.

Dans certains modes de réalisation, le premier capteur est une caméra (par exemple, une caméra main, une caméra de dermatoscope, une caméra de lampe à fente, une caméra de laryngoscope, etc.), un échographe, un électrocardiographe, un électroencéphalographe, un spiromètre, un tensiomètre, un thermomètre, un oxymètre, une balance, un réfracteur, un tonomètre, un pachymètre, un frontofocomètre, un kératomètre, un autoréfractomètre ou un appareil de radiologie comme un scanner, un appareil d'imagerie par résonance magnétique (IRM) ou un appareil de tomographie par émission de positions (ou PET scan), cet appareil de radiologie pouvant être couplé à un système d'archivage et de transmission d'image ou "PACS" (pour "Picture Archiving and Communication System").

La présente invention concerne également un système comprenant la station patient précitée et une station médecin, la station médecin comprenant une unité de traitement informatique configurée pour recevoir le signal vidéo de sortie transmis par la station patient.

Selon un deuxième aspect, la présente invention concerne un procédé d'acquisition et de transmission de données patient vers une station médecin distante via un réseau de télécommunications, le procédé comprenant les étapes suivantes :
- générer un premier signal vidéo ou une première image à partir de premières données patient issues d'un premier capteur d'un premier type et afficher le premier signal vidéo ou la première image sur un dispositif d'affichage principal dans une première fenêtre d'affichage ;
- capturer au moins une zone d'affichage de la première fenêtre d'affichage pour générer un signal vidéo capturé ; et
- transmettre un signal vidéo de sortie comprenant le signal vidéo capturé vers la station médecin via le réseau de télécommunication.

Dans certains modes de mise en œuvre, le procédé comprend en outre :
- générer un deuxième signal vidéo à partir de deuxièmes données patient issues d'un deuxième capteur d'un deuxième type ; et
- générer le signal vidéo de sortie par combinaison du deuxième signal vidéo et du signal vidéo capturé.

Les caractéristiques et avantages précités, ainsi que d'autres, apparaîtront à la lecture de la description détaillée qui suit, d'exemples de réalisation de la station patient et du procédé proposés. Cette description détaillée fait référence aux dessins annexés.

### BREVE DESCRIPTION DES DESSINS

Les dessins annexés sont schématiques. Ils visent avant tout à illustrer les principes de l'invention.

Sur ces dessins, d'une figure (FIG) à l'autre, des éléments (ou parties d'élément) identiques ou analogues sont repérés par les mêmes signes de référence.
- La FIG. 1 est un schéma général représentant un exemple de système de téléconsultation ;
- Les FIGS. 2A-2D représentent des exemples de station patient pouvant être utilisées sur le site patient lors d'une téléconsultation ;
- La FIG. 3 représente un schéma de fonctionnement d'un exemple de station patient ;
- La FIG. 4 représente un schéma de fonctionnement d'un autre exemple de station patient dont l'unité de traitement comprend une unité principale et une unité auxiliaire ;
- Les FIGS. 5A-5B illustrent différents exemples d'affichage de signaux vidéo sur le dispositif d'affichage de la station patient.

### DESCRIPTION DETAILLEE

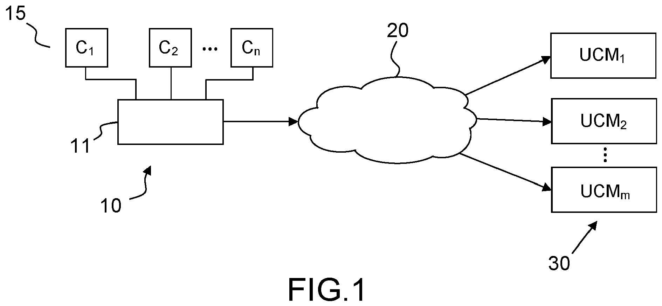

La FIG. 1 illustre un système de téléconsultation dans lequel des données patient recueillies par une station patient 10 sur un site patient sont transmises via un réseau de télécommunication 20 vers une ou plusieurs sites médecin 30 distants. La station patient 10 comprend une unité de traitement 11 à laquelle sont connectés un ensemble de capteurs C1, C2, ... Cn de différents types. L'unité de traitement 11 est en général un ordinateur comme, par exemple, un ordinateur personnel de bureau, un ordinateur portable, une tablette ou un smartphone. Les capteurs C1 à Cn peuvent par exemple comprendre des capteurs images ou des capteurs audio qui sont utilisables pour permettre l'examen clinique du patient. Selon la présente description, un capteur image est entendu comme un capteur qui produit des données susceptibles de générer des signaux de différents types tels que des vidéos, photos, images, images animés, données alphanumériques, ou données graphiques. Selon la présente description, un capteur audio est un capteur qui produit des données sous un format audio. A titre d'exemple les capteurs images peuvent être une caméra de type « webcam », une caméra main, une caméra de dermatoscope, une caméra de lampe à fente, une caméra de laryngoscope, un échographe, un électrocardiographe, un électroencéphalographe, un spiromètre, un tensiomètre, un thermomètre, un oxymètre, une balance, un réfracteur, un tonomètre, un pachymètre, un frontofocomètre, un kératomètre, un autoréfractomètre et/ou un appareil de radiologie. A titre d'exemple, les capteurs audio peuvent être un microphone et/ou un stéthoscope.

L'unité de traitement 11 est connectée à un réseau de télécommunication 20, par exemple le réseau internet, et peut donc transmettre et recevoir des flux d'informations, notamment des signaux vidéo et audio, lors de sessions de vidéoconférence avec des unités de traitement UCM1 à UCMm, appelées ci-après "unité de traitement médecin", localisées sur un ou plusieurs sites médecin 30 utilisées par les médecins et également connectées au réseau de télécommunication 20 comme le réseau internet. Les unités de traitement peuvent se connecter au réseau internet par différents moyens : Ethernet, Wifi, modem 3G/4G/5G, modem satellite, etc...

Bien que la téléconsultation soit en général effectuée depuis un site patient vers un site médecin 30 comprenant une unité de traitement médecin UCM1, il est aussi possible de transmettre les données patient depuis le site patient vers une pluralité de sites médecins 30, chaque site médecin comprenant une unité de traitement médecin UCM1 à UCMm.

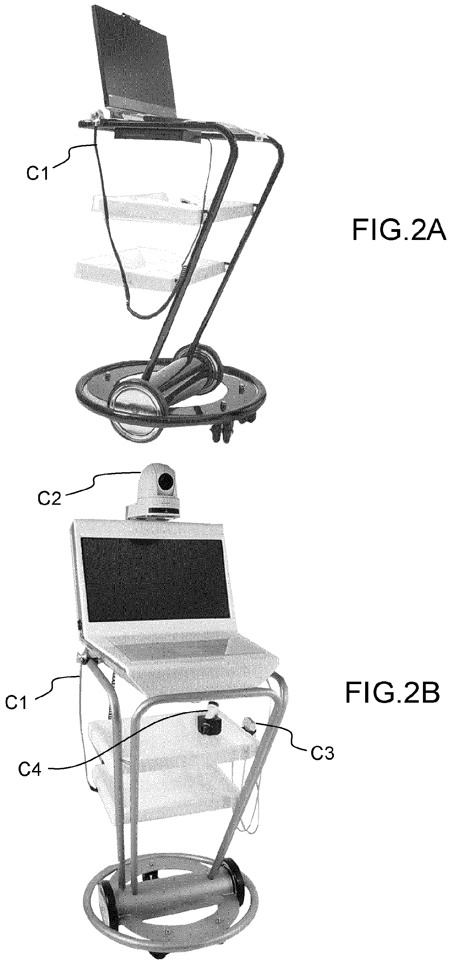

Les FIGS. 2A-2D représentent des exemples de station patient 10. Ces stations patient sont installées sur le site où le patient peut se situer comme, par exemple, dans un studio de téléconsultation, dans une pharmacie, dans un établissement médico-social, à l'hôpital, dans un cabinet de médecin, sur le lieu de travail ou au domicile du patient, etc...

La FIG. 2A est un exemple de station patient 10 de type chariot de téléconsultation. Il comprend un ordinateur personnel avec écran tactile orientable, une webcam, un système de microphone et d'enceinte, un clavier, un pavé tactile, un lecteur de carte d'identification (pour identifier le patient et/ou l'utilisateur de la station patient), un ensemble de capteurs, le tout étant supporté par une structure métallique montée sur roues avec un plateau support pour l'ordinateur et des bacs de rangement pour les capteurs. Un capteur C1 de type stéthoscope est visible sur la FIG.2A.

La FIG. 2B est un exemple de station patient 10 de type chariot de téléconsultation. Il comprend un ordinateur avec écran tactile orientable, une caméra orientable (aussi appelée caméra PTZ d'après l'acronyme anglais de « pan tilt zoom »), un système de microphone et d'enceinte, un clavier capacitif positionné sous une dalle de verre, un système de désinfection des mains par brumisation d'une solution hydroalcoolique, une batterie d'alimentation, un système de recharge électrique avec connexion magnétique, un lecteur de carte d'identification, le tout étant supporté par une structure métallique montée sur roues avec un plateau support pour l'ordinateur et des bacs de rangement pour les capteurs. Dans l'exemple de la FIG.2B on peut voir un capteur C1 de type stéthoscope, un capteur C2 formé par la caméra orientable, un capteur C3 de type échographe et un capteur C4 de type otoscope.

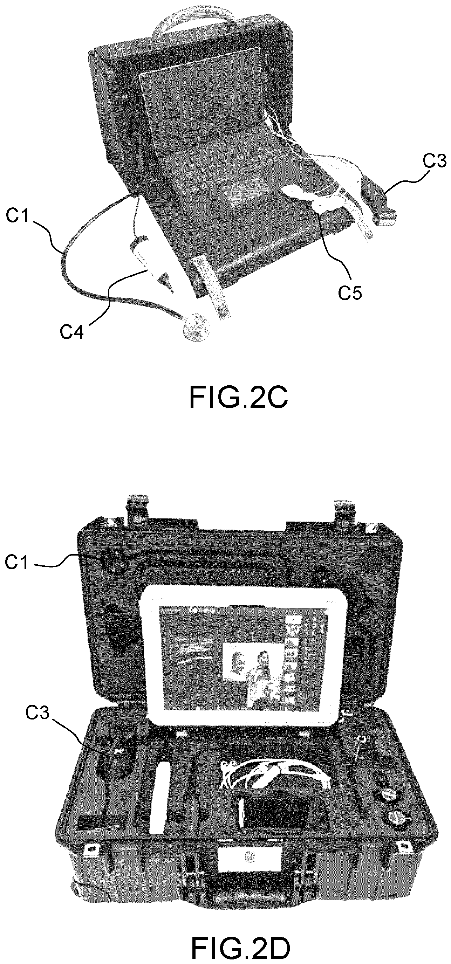

La FIG. 2C est un exemple de station patient 10 de type mallette de téléconsultation. Elle comprend un ordinateur avec écran tactile orientable de type « ordinateur-tablette », une webcam, un système de microphone et d'enceinte, un clavier, un pavé tactile, une batterie d'alimentation, un système de recharge électrique, un lecteur de carte d'identification, le tout étant supporté par une structure en plastique avec des filets intérieurs pour le rangement des capteurs. Dans l'exemple de la FIG. 2C on peut voir un capteur C1 de type stéthoscope, un capteur C3 de type échographe, un capteur C4 de type otoscope et un capteur C5 de type électrodes d'électrocardiographe.

La FIG. 2D est un exemple de station patient 10 de type mallette de téléconsultation. Elle comprend un ordinateur avec écran tactile orientable de type « ordinateur-tablette » (renforcé pour résister à environnement rigoureux), une webcam, un système de microphone et d'enceinte, un clavier, un pavé tactile, une batterie d'alimentation, un système de recharge électrique, un lecteur de carte d'identification, le tout étant supporté par une structure en plastique conforme à plusieurs normes (par exemple, la norme de type « Stanag » et l'indice de protection de type « IP67 ») avec un aménagement intérieur en mousse pour le rangement des capteurs. Dans l'exemple de la FIG.2C on peut voir notamment un capteur C1 de type stéthoscope et un capteur C3 de type échographe.

D'autres types de station patient 10 possibles, non représentées, comprennent des unités de traitement informatique de type tablettes ou de type smartphone.

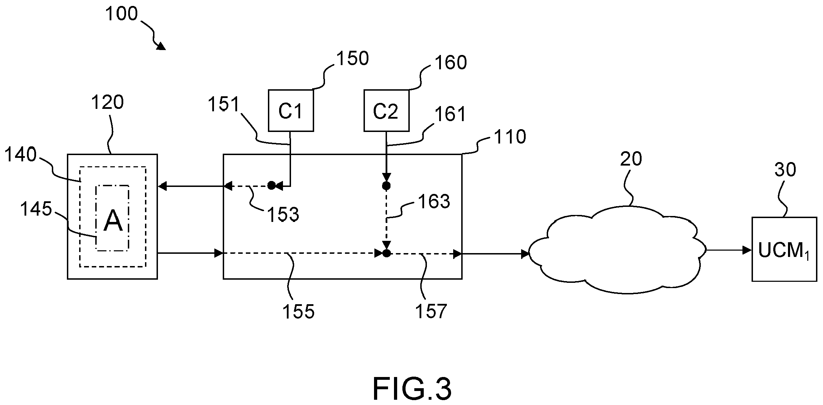

La FIG.3 représente un schéma de fonctionnement d'un système de téléconsultation comprenant un exemple de station patient 100 selon l'invention. La station patient 100 comprend un premier capteur 150 d'un premier type fournissant des premières données patient 151, un deuxième capteur 160 d'un deuxième type fournissant des deuxièmes données patient 161, une unité de traitement 110 qui reçoit les premières et deuxièmes données patient 151, 161, et un dispositif d'affichage 120. Les capteurs 150, 160, sont connectés à l'unité de traitement par une liaison filaire (e.g. un câble USB) ou une liaison sans fil (e.g. une liaison utilisant des ondes radio comme une liaison "Bluetooth")

L'unité de traitement 110 est en général dotée d'un système d'exploitation permettant d'exécuter des logiciels, en particulier des logiciels de téléconsultation, des logiciels de vidéoconférence, et des logiciels dédiés à des capteurs permettant d'afficher des signaux vidéo (ou images) fournis par des capteurs dans des fenêtres d'affichage, notamment au sein d'interfaces graphiques.

Selon l'exemple représenté sur la FIG.3, l'unité de traitement 110 est configurée pour générer un premier signal vidéo 153 ou une première image à partir des premières données patient 151 provenant du premier capteur 150. Le premier signal vidéo 153 est affiché dans une première fenêtre d'affichage 140 dédiée au capteur 150. Un logiciel avec une fonction de capture d'écran, communément appelé «SCR» selon l'acronyme en langue anglaise de « screen capture recorder », est exécuté par le système d'exploitation afin d'enregistrer une partie du premier signal vidéo 153 affiché dans la première fenêtre 140. Le logiciel de capture d'écran permet de capturer une zone d'affichage 145 de la première fenêtre d'affichage 140 dans laquelle le signal vidéo 153 affiché va être enregistré. Pour cela, il est possible de choisir plusieurs paramètres comme la taille et/ou la position de la zone d'affichage 145 à capturer. La fonction de capture d'écran permet ainsi d'obtenir un signal vidéo capturé 155 à partir du premier signal vidéo 153 issu des premières données patient 151 provenant du premier capteur 150.

Cette configuration est particulièrement avantageuse dans le cas où le signal vidéo 153 ne peut pas être directement transmis vers le réseau 20 par l'unité de traitement 110 et peut seulement être affiché sur le dispositif d'affichage 120 dans une première fenêtre d'affichage 140 liée au logiciel dédié au premier capteur 150, souvent fourni par le constructeur du capteur. Un tel capteur peut notamment être une caméra de type caméra de vidéosurveillance (communément appelée « caméra IP »), un otoscope, un échographe ou un électrocardiographe.

Dans l'exemple de la FIG. 3, la zone d'affichage 145 est incluse à l'intérieur de la première fenêtre d'affichage 140, cependant il est tout à fait possible de choisir une zone d'affichage 145 de la même taille que la fenêtre d'affichage 140, ou même d'une taille plus grande que la fenêtre d'affichage 140.

Selon une variante, la fonction de capture d'écran permet d'obtenir un signal vidéo capturé 155 à partir d'une première image issue de données patient provenant du premier capteur 150. La première image est d'abord affichée dans la première fenêtre d'affichage 140, puis elle est capturée pour générer le signal vidéo capturé 155. Ainsi la fonction de capture d'écran permet de transformer en signal vidéo une image issue d'un capteur par enregistrement vidéo de cette image. Selon d'autres variantes, il est possible d'utiliser la fonction de capture d'écran de la station patient 100 pour transformer en signal vidéo de sortie 153 une photographie, des données alphanumériques, ou des données graphiques. Ceci s'avère utile pour des capteurs pouvant fournir des images et/ou des graphiques comme les échographes et les électrocardiographes.

La génération du signal vidéo capturé 155 peut aussi comprendre une étape de filtrage, par exemple un rééchantillonnage ou un redimensionnement pour définir un format du signal vidéo capturé 155. En particulier, il est possible de rogner certaines zones du signal (suppression de bandes verticales ou horizontales de la vidéo par exemple). Dans la présente description, un format de signal vidéo comprend notamment la résolution du signal (généralement exprimé en pixels par pixels), l'encodage numérique (par exemple RGB 24bits) et le débit vidéo (généralement exprimé en nombre d'images par seconde).

Le signal vidéo capturé 155 est ensuite transmis en tant que signal vidéo de sortie 157 vers le réseau 20 par un logiciel de vidéoconférence. Le logiciel utilisé peut aussi être un logiciel de téléconsultation avec une fonction de vidéoconférence. Pour cela, le système d'exploitation exécute, par exemple, un logiciel de type pilote de périphérique (aussi appelé « driver ») qui fait en sorte que le signal vidéo de sortie 157 soit reconnu comme une source vidéo « normale» susceptible d'être transmise vers le réseau comme le serait un signal vidéo issu d'un dispositif de type webcam. En pratique, le driver virtuel indique au système d'exploitation que le signal vidéo de sortie 157 provient d'un périphérique de type caméra numérique, comme une webcam, alors que ce périphérique n'existe pas en réalité (i.e. ce périphérique est "virtuel"). En général, le signal vidéo de sortie 157 correspondant à la capture d'écran est alors sélectionnable par l'utilisateur dans un menu déroulant de l'interface graphique du logiciel de vidéoconférence qui permet la sélection des signaux vidéos, tout comme le serait le signal provenant d'une webcam connectée à l'unité de traitement 110.

Dans certains cas, le logiciel de vidéoconférence peut appliquer un traitement au signal de vidéo de sortie 157 avant de le transmettre à la station médecin 30 via le réseau 20 afin de modifier le format du signal vidéo de sortie 157. La nature des traitements appliqués peut être, par exemple, de la compression vidéo (notamment de la compression de type H264, Mpeg2, Mpeg4, H263, VP8, VP9), un changement de résolution, un changement de précision ou une paquétisation du signal pour faciliter le transport des signaux par un protocole internet utilisé par le réseau.

Selon d'autres exemples, également illustrés sur la FIG. 3, il est possible de combiner le signal vidéo capturé 155 avec un deuxième signal vidéo 163 issu de deuxièmes données patient 161 provenant d'un deuxième capteur 160. Ceci est en particulier utile lorsque le deuxième capteur 160 est une webcam et que l'on souhaite combiner le signal provenant de cette webcam et un signal provenant de la capture d'une fenêtre affichant le signal du premier capteur 150. On peut penser, par exemple, à la combinaison d'un signal provenant d'une webcam et d'un signal provenant d'un échographe nécessitant l'utilisation d'un logiciel dédié.

La combinaison peut être effectuée de différentes manières. En particulier il est possible de produire un signal vidéo de sortie 157 qui est un signal combiné spatialement à l'aide d'un mélangeur vidéo (communément appelé « mixer » en langue anglaise). Un tel signal correspond, une fois affiché sur un dispositif d'affichage, à une vidéo comprenant plusieurs parties juxtaposées. La combinaison permet alors d'obtenir un signal vidéo de sortie 157 unique qui peut être transmis, et reçu, par un logiciel de vidéoconférence classique.

Dans l'exemple de la FIG.3, uniquement deux capteurs 150, 160 sont connectés à la station patient 100, cependant il est possible d'avoir un plus grand nombre de capteurs connectés à la station patient 100. Dans ce cas, il est possible de combiner plus de deux signaux issus de données provenant de ces capteurs par la méthode de combinaison précitée.

Selon des variantes de l'invention, les capteurs comprennent des capteurs audio dont les données peuvent être traitées par l'unité de traitement 110 afin de générer des signaux audio, par exemple des capteurs comme un stéthoscope ou un microphone (un micro-casque connectable à la station patient par une connectique de type « jack 3,5mm » entre aussi dans ce type de capteurs). De la même manière que pour les signaux vidéo, il est possible de combiner plusieurs signaux audios différents en un signal audio de sortie unique au moyen de l'unité de traitement 110. La combinaison des signaux audio peut être effectuée par un mélangeur audio. Il est de plus possible d'appliquer des filtrages à un ou plusieurs signaux audio avant d'effectuer la combinaison, en particulier un filtrage de type égalisation de fréquence (notamment à l'aide d'un filtre communément appelé «equalizer» en langue anglaise) afin d'amplifier ou d'atténuer certaines bandes de fréquences sonores.

De la même manière que pour le signal vidéo de sortie 157, le système d'exploitation de l'unité de traitement 110 peut exécuter un logiciel de type pilote de périphérique adapté aux capteurs audio, afin de faire en sorte que le signal audio de sortie soit reconnu par le système d'exploitation comme un signal audio provenant d'un périphérique audio classique de type microphone. Ceci permet alors de sélectionner le signal audio de sortie en tant que signal audio qui sera utilisé par le logiciel de vidéoconférence pour la transmission vers le réseau. La sélection peut en pratique être faite à l'aide du menu déroulant de l'interface graphique du logiciel de vidéoconférence permettant de sélectionner les signaux audios.

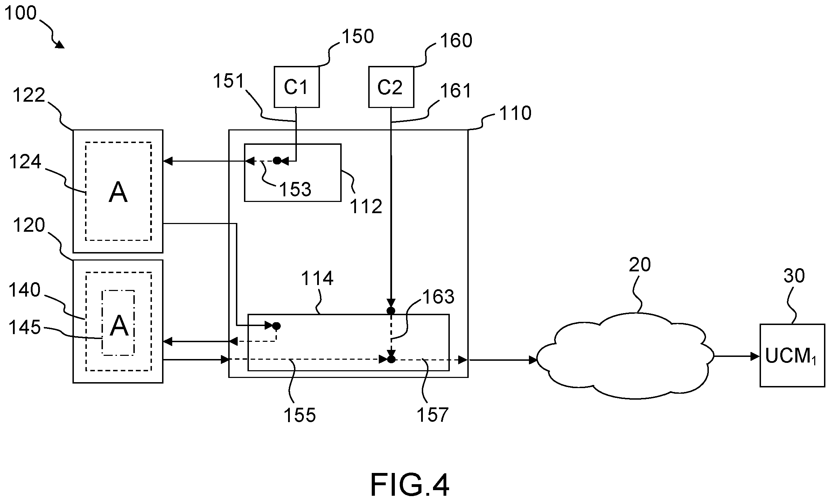

La FIG.4 illustre un autre exemple de station patient 100 dans laquelle l'unité de traitement 110 comprend deux unités distinctes : une unité auxiliaire 112 et une unité principale 114.

L'unité auxiliaire 112 est utilisée notamment pour générer le premier signal vidéo 153 ou la première image tandis que l'unité principale 114 est utilisée notamment pour la fonction de vidéoconférence.

L'unité auxiliaire 112 est configurée pour afficher le premier signal vidéo 153 issu des premières données patient 151 provenant du premier capteur 150 sur un dispositif d'affichage auxiliaire 122 dans une fenêtre d'affichage 124, également appelée fenêtre d'affichage auxiliaire.

En outre, le système d'exploitation de l'unité principale 114 est configuré pour exécuter un logiciel de «mise en miroir» (en anglais "screen mirroring") visant à dupliquer la fenêtre d'affichage auxiliaire 124 sur le dispositif d'affichage principal 120. Ainsi le premier signal vidéo 153 est affiché dans une fenêtre d'affichage 140 du dispositif d'affichage principal 120, et une zone d'affichage 145 de la fenêtre 140 peut ensuite être capturée par le système décrit plus haut afin de générer un signal vidéo capturé 155. Dans cet exemple, il est également possible de combiner le signal vidéo capturé 155 avec un deuxième signal vidéo 163 issu de deuxième données patient 161 provenant d'un deuxième capteur 160 au moyen de l'unité principale 114.

Le logiciel de mise en miroir peut effectuer la duplication de l'affichage 124 en échangeant des données entre l'unité auxiliaire 112 et l'unité principale 114 par différents moyens et, par exemple, via un réseau de communication de type wifi auquel sont connectés l'unité auxiliaire 112 et l'unité principale 114. Le signal vidéo issu de duplication est en général affiché sur le dispositif d'affichage principal 120 au moyen d'un logiciel dédié.

Cette configuration permet d'utiliser la station patient 100 avec des capteurs fournis avec un logiciel dédié qui est compatible avec le système d'exploitation utilisé dans l'unité auxiliaire 112 mais n'est pas compatible avec le système d'exploitation utilisé dans l'unité principale 114. Une caméra de téléphone multifonction (ou smartphone) entre par exemple dans cette catégorie. En effet, la caméra intégrée du smartphone est compatible avec le système d'exploitation du téléphone (qui est considéré comme une unité auxiliaire) mais la caméra intégrée du téléphone n'est pas reconnue comme caméra ou webcam par le système d'exploitation de l'unité principale 114. Une caméra de surveillance ou tout autre type de capteur dont la vidéo produite n'est affichable que via un logiciel exécuté sur un téléphone ou un autre dispositif analogue entre également dans cette catégorie.

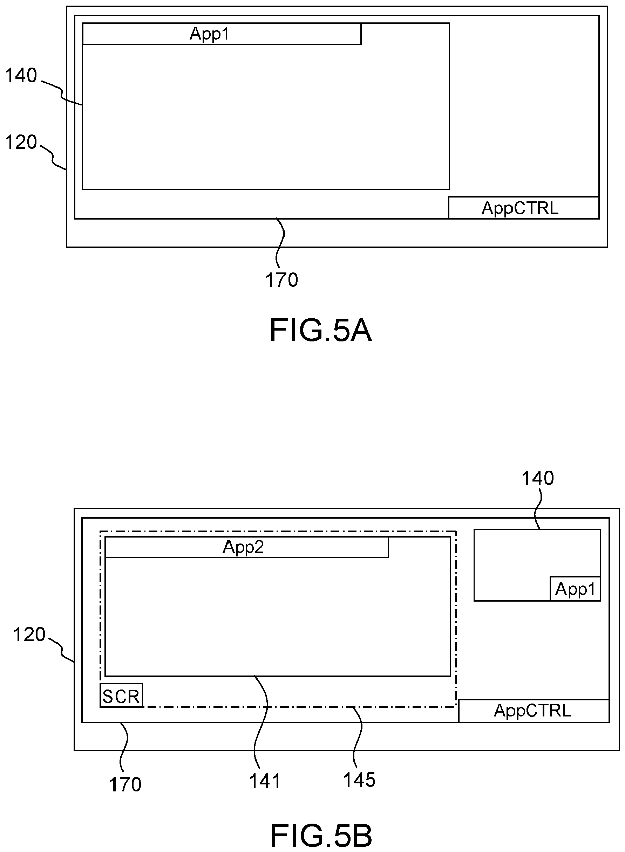

Les FIGS. 5A-5B illustrent l'affichage sur le dispositif d'affichage 120 de la station patient 100 au cours d'un téléconsultation selon plusieurs exemples d'utilisation.

Selon un exemple, un logiciel de téléconsultation AppCTRL est exécuté sur la station patient 100 afin de mettre en œuvre une téléconsultation. Le logiciel de téléconsultation est exécuté par l'unité de traitement 110 et présente en général une interface graphique de contrôle 170 qui est affichée sur le dispositif d'affichage 120.

Un utilisateur de la station patient 100, qui peut être un assistant de consultation ou le patient lui-même, peut interagir avec le logiciel de téléconsultation via son interface graphique de contrôle 170 en utilisant des périphériques classiquement connectés à l'unité de traitement 110, comme une souris, un clavier, un écran tactile ou un pavé tactile.

L'utilisateur de la station patient 100 peut alors activer un capteur en lançant un logiciel dédié App1, c'est-à-dire qu'il peut par exemple utiliser une webcam en lançant le logiciel de visualisation dédié à la webcam. Il est aussi possible d'activer plusieurs capteurs au moyen de plusieurs logiciel dédiés. L'activation d'un ou plusieurs capteurs peut aussi être effectuée automatiquement lors du démarrage du système d'exploitation de l'unité de traitement 110.

Selon l'exemple illustré sur la FIG.5A, l'unité de traitement 110 peut faire en sorte que la fenêtre d'affichage 140 contenant les signaux vidéo ou images provenant du capteur (par exemple de la webcam) s'affiche à l'intérieur de l'interface graphique de contrôle 170. Ainsi, il est possible de contrôler la première fenêtre d'affichage 140 par l'intermédiaire de l'interface graphique de contrôle 170. En particulier, si la fenêtre de l'interface graphique de contrôle 170 est réduite en taille via une action volontaire de l'utilisateur (e.g. avec la souris connectée à l'unité de traitement 110), alors la fenêtre 140 du logiciel dédié App1 qui est intégrée à l'intérieur de l'interface graphique de contrôle 170, verra sa taille se réduire également, de façon proportionnelle.

L'affichage de la fenêtre d'affichage 140 d'un logiciel App1 à l'intérieur de l'interface graphique de contrôle 170, qui peut être vu comme une «prise de contrôle» de la fenêtre d'affichage 140 du logiciel App1 dédié au capteur par le logiciel de téléconsultation AppCTRL, peut être effectué par différent moyens. En particulier, il est possible de programmer la plateforme de téléconsultation dans un environnement de développement de type « C++ Qt » et d'utiliser des fonctions de prise de contrôle connues comme « QWindow::fromWinId » et « QWidget::createWindowContainer ».

Par ailleurs, si le logiciel App1 dédié au capteur est de nature à être exécuté au sein d'un navigateur internet, c'est-à-dire que la fenêtre d'affichage du logiciel App1 s'affiche à l'intérieur de la fenêtre d'affichage du navigateur internet, alors le logiciel de téléconsultation AppCTRL prendra le contrôle du navigateur internet au sein duquel le logiciel App1 est exécuté. Ceci est rendu possible, par exemple, en utilisant l'environnement de développement de type « C++ Qt » qui permet de mettre en œuvre un navigateur internet dans un autre logiciel au moyen d'un moteur de rendu, aussi appelé de manière générique « web engine » en langue anglaise, et plus spécifiquement appelé « Webkit » ou « QTwebengine » dans le cas d'un environnement de développement de type « C++ Qt ».

En pratique, si le logiciel App1 dédié au capteur est de nature à être exécuté au sein d'un navigateur internet, le logiciel de téléconsultation AppCTRL injecte l'adresse internet du logiciel App1 dans le navigateur internet (qui est intégré dans AppCTRL au moyen du moteur de rendu) et AppCTRL affiche alors la fenêtre d'affichage du navigateur internet dans son interface graphique 170.

Selon une variante illustrée dans la FIG. 5B, lorsqu'un utilisateur lance un deuxième logiciel App2 dédié à un deuxième capteur alors qu'un premier logiciel App1 dédié à un premier capteur est déjà en cours d'utilisation, alors l'unité de traitement 110 fait en sorte de redimensionner automatiquement la première fenêtre d'affichage 140 (du logiciel App1) afin de laisser apparaître intégralement le nouvelle fenêtre d'affichage 141 (du logiciel App2). Ceci dans le but que les deux fenêtres 140, 141 soient visibles simultanément à l'intérieur de l'interface graphique de contrôle 170 sur le dispositif d'affichage 120. Cette variante est particulièrement avantageuse lorsque l'utilisateur veut visualiser des signaux vidéo de deux capteurs simultanément.

Par ailleurs, il est alors possible d'exécuter un logiciel de capture d'écran SCR afin de capturer une zone d'affichage 145 de la fenêtre d'affichage 141 du logiciel App2. Cette variante est particulièrement avantageuse lorsque l'utilisateur veut visualiser des signaux vidéo issus de deux capteurs simultanément et que l'un des capteurs nécessite l'utilisation d'une fonction de capture d'écran pour la transmission des données vers le réseau.

Par exemple, il est ainsi possible de visualiser sur le dispositif d'affichage 120, l'image médecin provenant d'une webcam affichée dans une première fenêtre 140, tout en effectuant une capture d'une zone d'affichage 145 de la fenêtre 141 du logiciel App2.

Bien que dans l'exemple de la FIG.5B la zone d'affichage 145 capturée englobe la fenêtre d'affichage 141 du logiciel App2, selon d'autres exemples il est possible que la zone d'affichage 145 capturée ne recouvre qu'une partie de la fenêtre d'affichage 141.

Selon d'autres variantes de l'invention, il est possible de définir et sauvegarder des profils utilisateur dans une mémoire de stockage de l'unité de traitement 110. Ces profils constituent un ensemble d'instructions permettant d'activer un certain nombre de capteurs et leur logiciel dédié respectif, de définir le format des signaux vidéo générés par l'unité de traitement 110, d'ordonner ou non le lancement d'un ou plusieurs logiciels de capture d'écran (et de définir la taille et position des zones d'affichages à capturer), ou encore de définir la nature des filtrages à appliquer aux signaux vidéo et/ou audio issus des divers capteurs.

Lorsque l'examen clinique à réaliser lors d'une téléconsultation est d'un certain type, par exemple un examen des canaux auditifs, alors l'utilisateur peut sélectionner un profil adéquat qui activera les capteurs nécessaires à l'examen, par exemple une webcam et un otoscope.

En outre, les profils pourront servir à définir les paramètres concernant le redimensionnement automatique de certaines fenêtres d'affichage 141 au sein de l'interface graphique de contrôle 170 lors de l'utilisation simultanée de plusieurs capteurs.

Enfin, un profil peut être défini par défaut, par exemple un profil de base permettant d'effectuer simplement une vidéoconférence. Le profil par défaut sera appliqué dès le démarrage de l'unité de traitement 110. D'autres profils définis au préalable peuvent être appliqués en cours de session de téléconsultation par l'utilisateur de la station patient 100, par exemple par clic de souris ou bien via une possible fonction tactile du dispositif d'affichage 120.

Les profils peuvent aussi servir à définir le format vidéo et/ou audio à utiliser pour le signal vidéo de sortie et/ou le signal audio de sortie. Les profils peuvent être enregistrés dans la mémoire de l'unité de traitement sous forme d'un fichier (par exemple un fichier au format .xml ou .json) ou bien dans une base de données accessible par un réseau. Avantageusement, tant que deux profils différents définissent le même format de signal vidéo de sortie et le même format de signal audio de sortie, il est possible de passer de l'un à l'autre en cours de téléconsultation sans problème, car ceci ne perturbe pas le fonctionnement d'un logiciel de vidéoconférence classique (qui n'accepte en général pas de changement de format de signaux en cours de vidéoconférence).

Les modes ou exemples de réalisation décrits dans la présente invention sont donnés à titre illustratif et non limitatif, une personne du métier pouvant facilement, au vu de cette invention, modifier ces modes ou exemples de réalisation, ou en envisager d'autres, tout en restant dans la portée de l'invention. En particulier, une personne du métier pourra facilement envisager des variantes ne comprenant qu'une partie des caractéristiques des modes ou exemples de réalisation précédemment décrits, si ces caractéristiques à elles seules suffisent pour procurer un des avantages de l'invention. De plus, les différentes caractéristiques de ces modes ou exemples de réalisation peuvent être utilisées seules ou être combinées entre elles. Lorsqu'elles sont combinées, ces caractéristiques peuvent l'être comme décrit ci-dessus ou différemment, l'invention ne se limitant pas aux combinaisons spécifiques décrites dans la présente invention. En particulier, sauf précision contraire, une caractéristique décrite en relation avec un mode ou exemple de réalisation peut être appliquée de manière analogue à un autre mode ou exemple de réalisation.

## Revendications

1. Station patient (100) pour la télémédecine permettant d'acquérir des données relatives à un patient et de transmettre ces données vers une station médecin (30) distante, via un réseau de télécommunication (20), la station patient (100) comprenant :
- une unité de traitement informatique (110) ;
- un dispositif d'affichage principal (120) ; et
- au moins un premier capteur (150) d'un premier type générant des premières données patient (151) ;
dans laquelle l'unité de traitement (110) est configurée pour :
- générer un premier signal vidéo (153) ou une première image à partir des premières données patient (151) et afficher le premier signal vidéo (153) ou la première image sur le dispositif d'affichage principal dans une première fenêtre d'affichage (140) ;
- capturer au moins une zone d'affichage (145) de la première fenêtre d'affichage (140) pour générer un signal vidéo capturé (155) ; et
- transmettre un signal vidéo de sortie (157) comprenant le signal vidéo capturé (155) vers la station médecin (10) via le réseau de télécommunication (20).

2. Station patient (100) selon la revendication 1, comprenant en outre :
- au moins un deuxième capteur (160) d'un deuxième type, différent du premier type, générant des deuxièmes données patient (161) ;
dans laquelle l'unité de traitement (110) est configurée pour :
- générer un deuxième signal vidéo (163) à partir des deuxièmes données patient (161) ; et
- générer le signal vidéo de sortie (157) par combinaison du deuxième signal vidéo (163) et du signal vidéo capturé (155).

3. Station patient selon la revendication 1 ou 2, dans laquelle l'unité de traitement (110) est associée à un système d'exploitation et dans laquelle l'unité de traitement (110) est configurée pour que le signal vidéo de sortie (157) soit identifié par le système d'exploitation comme un signal vidéo émanant d'une caméra numérique connectée à l'unité de traitement (110).

4. Station patient selon l'une quelconque des revendications 1 à 3, dans laquelle la transmission du signal vidéo de sortie (157) vers la station médecin (10) est réalisée par l'intermédiaire d'un logiciel de vidéoconférence, l'unité de traitement (110) étant configurée pour que le signal vidéo de sortie (157) soit identifié par le logiciel de vidéoconférence comme un signal vidéo émanant d'une caméra numérique connectée à l'unité de traitement (110).

5. Station patient selon l'une quelconque des revendications précédentes, dans laquelle l'unité de traitement (110) est configurée pour afficher la première fenêtre d'affichage (140) à l'intérieur d'une deuxième fenêtre d'affichage d'une interface graphique de contrôle.

6. Station patient selon la revendication 5, dans laquelle l'unité de traitement (110) est configurée pour redimensionner automatiquement la première fenêtre d'affichage (140) affichée dans l'interface graphique de contrôle.

7. Station patient selon l'une quelconque des revendications 1 à 6, dans laquelle la première fenêtre d'affichage (140) fait partie d'une interface graphique dédiée au premier capteur (150).

8. Station patient selon l'une quelconque des revendications 1 à 7, dans laquelle l'unité de traitement (110) comprend une unité principale (114) et une unité auxiliaire (112), l'unité auxiliaire (112) étant configurée pour générer le premier signal vidéo (153) ou la première image à partir des premières données patient (151) et afficher le premier signal vidéo (153) ou la première image sur un dispositif d'affichage auxiliaire (122); et l'unité principale (114) étant configurée pour dupliquer l'affichage (124) du premier signal vidéo (153) ou de la première image sur le dispositif d'affichage principal (120) dans la première fenêtre d'affichage (140).

9. Station patient selon l'une quelconque des revendications 1 à 8, dans laquelle la génération du signal vidéo de sortie (157) comprend une étape de filtrage et/ou de redimensionnement du signal vidéo capturé (155) et/ou du deuxième signal vidéo (163).

10. Station patient selon l'une quelconque des revendications 1 à 9, dans laquelle l'unité de traitement (110) est configurée pour transmettre le signal vidéo de sortie (157) vers la station médecin (10) lors d'une téléconsultation, et dans laquelle l'unité de traitement (110) est configurée, en outre, pour maintenir fixes des paramètres vidéo du signal vidéo de sortie (157), en particulier la résolution du signal vidéo de sortie, un débit du signal vidéo de sortie et/ou un type d'encodage numérique du signal vidéo de sortie, pendant la téléconsultation.

11. Station patient selon l'une quelconque des revendications précédentes dans lequel le premier capteur (150) est une caméra, un échographe, un électrocardiographe, un électroencéphalographe, un spiromètre, un tensiomètre, un thermomètre, un oxymètre, une balance, un réfracteur, un tonomètre, un pachymètre, un frontofocomètre, un kératomètre, un autoréfractomètre ou un appareil de radiologie.

12. Station patient selon l'une quelconque des revendications précédentes comprenant un capteur audio relié à l'unité de traitement et générant un premier signal audio, dans laquelle l'unité de traitement (110) est associée à un système d'exploitation et configurée pour que le premier signal audio soit identifié par le système d'exploitation comme un signal audio émanant d'un microphone connecté à l'unité de traitement (110).

13. Procédé d'acquisition et de transmission de données patient (161) vers une station médecin (30) distante via un réseau de télécommunications (20), le procédé comprenant les étapes suivantes:
- générer un premier signal vidéo (153) ou une première image à partir de premières données patient (151) issues d'un premier capteur (150) d'un premier type et afficher le premier signal vidéo (153) ou la première image sur un dispositif d'affichage principal (120) dans une première fenêtre d'affichage (140) ;
- capturer au moins une zone d'affichage (145) de la première fenêtre d'affichage (140) pour générer un signal vidéo capturé (155) ; et
- transmettre un signal vidéo de sortie (157) comprenant le signal vidéo capturé (155) vers la station médecin (10) via le réseau de télécommunication (20).

14. Procédé selon la revendication 13, comprenant en outre :
- générer un deuxième signal vidéo (163) à partir de deuxièmes données patient (161) issues d'un deuxième capteur (160) d'un deuxième type ; et
- générer le signal vidéo de sortie (157) par combinaison du deuxième signal vidéo (163) et du signal vidéo capturé (155).
